**Europäisches Patentamt**

**European Patent Office**

**Office européen des brevets**

(11) Veröffentlichungsnummer: **0 306 779**

**A2**

## (12) EUROPÄISCHE PATENTANMELDUNG

(21) Anmeldenummer: **88113927.3**

(22) Anmeldetag: **26.08.88**

(51) Int. Cl.⁴: **F26B 21/00 , F26B 9/06**

(30) Priorität: **09.09.87 DE 3730178**

(43) Veröffentlichungstag der Anmeldung:
**15.03.89 Patentblatt 89/11**

(84) Benannte Vertragsstaaten:
**AT BE CH DE ES FR GB GR IT LI NL SE**

(71) Anmelder: **Baltes, Hans**
**Heideweg 27**
**D-4600 Dortmund(DE)**

(72) Erfinder: **Baltes, Hans**
**Heideweg 27**
**D-4600 Dortmund(DE)**

(74) Vertreter: **Patentanwälte Schulze Horn und**
**Hoffmeister**
**Goldstrasse 36**
**D-4400 Münster(DE)**

(54) **Verfahren zum Trocknen, Lüften und Sterilisieren von Gut.**

(57) 1. Verfahren und Vorrichtung zum Trocknen, Lüften und Serilisieren von Gut.

2.1. Es sind Verfahren mit Hilfe von Trockenschränken bekannt, bei denen Luft mittels eines Gebläses nach dem Vorbeistreichen an Heizelementen in den Schrankinnenraum gedrückt wird um die Flüssigkeit im Trockengut aufnehmen zu können, und anschließend aus dem Schrankinnenraum geleitet wird.

Nachteilig sind die Energiekosten, der druckfeste Aufbau, die beträchtlichen Luftbewegungen und komplizierte Regelmechanismen. Es ist Aufgabe der Erfindung den Energieverbrauch zu reduzieren, wobei die Durchflußmasse der Luft möglichst gering gehalten wird. Auch die Herstellungskosten sollen gesenkt werden.

2.2. Die Aufgabe wird durch ein Verfahren mit einem Trockenschrank gelöst, bei dem das Gebläse die Luft aus dem Trockenschrankinnenraum saugt. Hierdurch sind Energieeinsparungen von 12 - 15% zu erzielen.

2.3. Mit Hilfe des Verfahrens kann Wäsche, empfindliches Gewebe, Wolle Oberbetten, Daunen, Leder, Bestecke, Instrumente, Kämme und Messer getrocknet und sterilisiert werden.

Fig.1

## Verfahren zum Trocknen, Lüften und Sterilisieren von Gut

Die Erfindung betrifft ein Verfahren und eine Vorrichtung zur Durchführung des Verfahrens zum Trocknen, Lüften und Sterilisieren von Gut, wie von Wäsche, besonders empfindlichen Geweben, reiner Wolle, Oberbetten, Kissen, Daunendecken, Wolldecken und Leder, festes Gut wie medizinische Instrumente, Bestecke, Kämme, Scheren, Bürsten, Messer und Rasierapparate, in einem Trockenschrank mit einem die Luft bewegenden Gebläse, wobei Außenluft durch eine Heizung erwärmt wird, danach einen Schrankinnenraum mit darin befindlichem Gut durchströmt und anschließend aus dem Trockenschrank durch eine Luftauslaßöffnung ausströmt.

Derartige Trockenverfahren und Trockenschränke sind bereits aus der DE-PS 26 46 903 bekannt. Ein derartiger Trockenschrank besteht aus doppelwandigen Seitenwänden, wobei die inneren Seitenwände Schlitze tragen, durch die aus dem durch die Doppelwandung gebildeten Lumen Luft in den Schrankinnenraum gelangen und von dort auch wieder abgeführt werden kann. In dem Schrankinnenraum befindet sich das zu trockenende Gut, welches in der Art aufgehängt ist, daß eine möglichst große freie Oberfläche auftritt. Der Innenraum ist weiterhin durch eine Rückwand und durch ein oberes und unteres Abschlußblech nach außen abgeschirmt. Die Frontpartie weist eine Tür auf, welche durch einen drehbaren Griff, der uber ein Gestänge Haken in Ausformungen der Seitenwände eingreifen läßt, zu verschließen ist.

Die Außenluft wird durch eine Luftansaugöffnung unterhalb des oberen Abdeckbleches durch ein Gebläse angesaugt und streicht anschließend an einem Heizelement entlang, um sich auf die erwünschte Temperatur zu erhitzen. Die Luft wird durch das Gebläse entlang dem Innenlumen einer durch die Doppelwandung gebildeten Zuleitung durch Schlitze in der einen inneren Seitenwand in den Schrankinnenraum gedruckt. Die bewegte luft wird anschließend an dem zu trocknenden Gut entlanggeführt, um auf der gegenüberliegenden Seite ebenfalls durch Schlitze in der anderen Innenseitenwand in das durch die Doppelwandung gebildete Lumen der Ableitung weitergeblasen zu werden. Die Ableitung führt zu einer Luftauslaßöffnung, durch die die Außenluft nach dem Aufheizen und nach dem Kontakt mit dem zu trocknenden Gut den Trockenschrank verläßt. Der durch das Geblase aufzwendende Druck muß ausreichend dimensioniert sein, um die auftretenden Reibungskräfte zu uberwinden. Daher ist es erforderlich, den Schrank mit einer drucksicheren Beschickungstür auszustatten, die mit einem ausreichend dimensionierten Verschlußmechanismus versehen sein muß.

Das Gebläse wird während seiner Arbeit durch die aus der Ansaugöffnung kommende, vorbeistreichende Außenluft gekühlt.

Derartige Trockner haben sich als sehr energiesparend erwiesen. Um die für die Trocknung erforderliche Verdunstung zu erzielen, wird eine große Luftmenge pro Zeiteinheit an dem zu trocknenden Gut vorbeigeführt, wobei sich zugleich die Heizleistung der Heizelemente in Grenzen halten. Trotz dieser Energieeinsparung ist es wünschenswert, den Energieverbrauch der Geräte weiterhin zu reduzieren. Es ist daher Aufgabe der Erfindung, die aufzuwendende Energie zu minimieren, die Verdunstung des Wassers in Relation zu der vorbeistreichenden Luft mög lichst effektiv zu gestalten und zugleich eine hohe Temperaturerhöhung der zu erwärmenden Außenluft zu erzielen, wobei die Durchflußmasse an Luft möglichst gering gehalten wird. Weiterhin sollen die Herstellungskosten gesenkt werden, indem auf aufwendige Konstruktionen, wie z. B. die Abdichtungen vom Gebläseaustritt über die Heizung in den Eintrittskanal, wie die Türverriegelung oder wie komplizierte Regelmechanismen, verzichtet werden.

Die Aufgabe wird erfindungsgemäß durch ein Verfahren der eingangs genannten Art gelöst, bei dem das Gebläse die Luft aus dem Trockenschrank absaugt. Bei den bisher hergestellten Geräten wurden Verfahren verwandt, bei denen ein Gebläse Luft durch eine Zuleitung in den Schrankinnenraum geblasen hat. Es wurde also mit Überdruck gearbeitet. Es besteht das technische Vorurteil, daß bei Trocknungsprozessen, in denen die Luft bewegt wird, die Luft entlang eines zu trocknenden Gutes unter Druck geblasen werden muß. Es ist üblich, zuerst das druckerzeugende Gebläse und anschließend das erforderliche Heizelement von der Luft anströmen zu lassen. Dagegen weist die Erfinung einen Trocknungsprozeß auf, bei dem mit Unterdruck gearbeitet wird. Die zur Trocknung erforderliche Luft wird durch die Zuleitung in den Schrankinnenraum gesogen und durch eine Ableitung zu einem ein Teilvakuum erzeugendes Gebläse gesaugt. Es besteht bisher die Vorstellung, daß eine entsprechende Saugbewegung nicht zu einer derartigen Trocknungsleistung in der Lage sei, wie es bei einem mit Überdruck arbeitenden Verfahren der Fall ist.

Das mit Unterdruck arbeitende Verfahren weist verschiedene bedeutsame Vorteile auf. Durch das Ansaugen der Luft ergibt sich in dem Schrankinnenraum ein Unterdruck, der verglichen mit dem bei Überdruck arbeitenden Verfahren eine beträchtliche Energieeinsparung bewirkt, die etwa bei 12 - 15 % liegt. Dieses ist dadurch zu begründen, daß eine geringere Masse an Luft beschleunigt und transportiert werden muß, als es bei einem Verfahren mit Überdruck der Fall ist. Wegen der geringeren bewegten Luftmassen ist auch die Masse an Trockenluft geringer, die zu erwärmen ist. Von besonderer Bedeutung ist der Effekt, daß das Wasser von der flüssigen in die gasförmige Phase leichter übergeht, je geringer der Gesamtdruck ist. Je geringer der Partialdruck der Luft ist, um so wirkungsvoller verläuft die Verdunstung des Wassers. Da der Partialdruck des Wassers bei einer vorgegebenen Temperatur konstant ist, spielt es keine Rolle, wie hoch der Druck der anderen, ebenfalls vorliegenden Gase ist. Inhalt der Erfindung ist es, den Übertritt von der flüssigen in die gasförmige Phase zu erleichtern, was letztendlich zu einem zügig ablaufenden und dadurch energieeinsparenden Verfahren führt. Weiterhin ist es wegen des konstanten Partialdruckes des Wassers bei einer vorgegebenen Temperatur erstrebenswert, ein möglichst großes Volumen an Luft auszutauschen, ohne jedoch auch zugleich große Massen zu bewegen. Die Lösung dieser Problematik besteht in dem Verfahren, die zur Trocknung verwandte Luft durch den Trockenschrank, d. h. längs des zu trocknenden Gutes, zu saugen und nicht wie bisher mittels eines Druckgebläses in den Schrank zu pumpen. Dabei wird innerhalb des Schrankes, d. h. im Schrankinnenraum durch das Absaugen ein Unterdruck gegenüber der Außenluft erzeugt, wodurch eine Druckdifferenz von bis zu 50 Pascal, vorzugsweise von 20 - 30 Pascal, entsteht.

Neben der Energieersparnis ist weiterhin von Vorteil, ein billigeres Herstellungsverfahren anwenden zu können, als es zuvor der Fall war. Bei einem erfindungsgemäßen Trockenschrank ist für das Verschließen der Tür nur noch ein schwach dimensionierter Verschluß erforderlich, da bei Inbetriebnahme der im Schrankinnenraum entstehende Unterdruck die Tür gegen den Türrahmen zieht. So sind einfach herzustellende Gummiwülste und Gummidichtungen ausreichend, um eine sichere Abdichtung zwischen Außen-und Innenluft erfolgen zu lassen. Eine mechanisch stabile und aufwendige Konstruktion ist nicht mehr erforderlich, was zu einer Senkung der Herstellungskosten führt.

Um die Temperatur der angesaugten Luft zu regulieren, können durch Veränderung der Förderleistung des Gebläses bei konstanter Leistung der Heizung unterschiedliche Durchflußmengen an Luft durch den Trockenschrank gesaugt werden. Eine derartige ungewöhnliche Temperaturregulierung ist im Zusammenhang mit dem erfindungsgemäßen Verfahren verständlich. Bei einem solchen Trokkenverfahren ist nicht die hohe Lufttemperatur das primäre Mittel, um eine Trocknung zu erzielen, sondern ein möglichst ausgeprägter, intensiver Luftaustausch ist das bevorzugte Mittel des Trokkenprozesses.

Bei einem solchen Verfahren wird zu Beginn des Trocknens bei voller Heizleistung die im Schrankinneren bewegte Luftmenge gering gehalten, indem das Gebläse nur eine geringe Förderleistung aufweist. Dabei ist die bewegte Luftmenge derart dimensioniert, daß der Schrankinnenraum samt Trockengut sich auf etwa 45° aufheizt. Diese Tempereatur wird dann über einen gewissen Zeitraum konstant gehalten, wobei die Heizleistung weiterhin maximal abläuft. Durch Variieren der Förderleistung des Gebläses wird die Temperatur reguliert. Wenn die Temperatur von 45° C überschritten wird, wird die Förderleistung des Gebläses angehoben. Der hohe Luftdurchsatz schafft wegen der der Luft zu entziehenden Verdunstungswärme bei feuchtem Gut eine Abkühlung des Schrankinnenraumes. Wird die Temperatur von 45° erreicht, wird das Gebläse auf eine geringe Leistung eingestellt. Der geringere Luftstrom führt folgerichtig zu einem geringeren Wärmeentzug durch Verdunstungswärme, so daß der Schrankinnenraum letztendlich sich wieder aufheizt. Wenn die Restfeuchtigkeit in dem Trockengut nicht mehr ausreichende Temperaturer niedrigungen durch die aufzwendende Verdunstungswärme auch bei maximaler Leistung des Gebläses hervorruft, heizt der Schrank sich allmählich auf höhere Temperaturen auf.

Der Vorteil einer Regulierung der Gebläseleistung besteht darin, daß lediglich geringe Ströme in Schaltungselementen reguliert werden müssen. Der Strombedarf des Ventilators (etwa 120 - 200 Watt) liegt etwa eine Zehnerpotenz unter dem Bedarf des Heizungselementes (etwa 2000 - 3300 Watt).

Ein Ausführungsbeispiel eines Trockenschrankes zur Durchführung des Verfahrens mit einer Luftansaugöffnung, mit einer von der Luftansaugöffnung kommenden, zu dem Schrankinnenraum führenden Zuleitung, wobei eine Heizung für die Trockenluft innerhalb der Zuleitung angeordnet ist, und mit einer von dem Schrankinnenraum wegführenden und mit einer Luftauslaßöffnung verbundenen Ableitung, ist derart gestaltet, daß das Gebläse innerhalb der Ableitung angeordnet ist.

Um den Motor stets gut kühlen zu können, wird in einer weiteren Ausführungsform des Trokkenschrankes vorgeschlagen, wobei das Gebläse aus auf einer gemeinsamen Welle liegendem Motor und Turbinenrad besteht, daß Motor und Turbinenrad durch eine im wesentlichen luftdichte Wand getrennt sind, die eine Durchführung für die Welle besitzt. Eine derartige Anordnung von Motor und Turbinenrad hat den Vorteil, daß unabhängig von den auftretenden Temperaturen der Motor stets bei Raumtemperatur gehalten wird. Zwar ist es bei der heutigen Technik kein Problem mehr, Motoren zu konstruieren, die Temperaturen über 100° C problemlos verkraften konnen, jedoch muß dieser Vorteil stets durch ein kostenaufwendigeres Herstellungsverfahren erkauft werden. Daher ist es für die Kostenbilanz bei der Herstellung eines erfindungsgemäßen Trok kenschrankes sinnvoll, den Motor des Gebläses nicht im direkten Kontakt mit der aus dem Schrankinnern kommenden Luft treten zu lassen. Bei einer derartigen Konstruktion ist es besonders vorteilhaft, wenn die durch die Luftansaugöffnung kommende Luft vor der Passage der Heizung im vollen Umfang an dem Motor vorbeistreicht. Eine solche Anordnung führt einerseits zu einer sehr effektiven Kühlung des Motors und andererseits wird die bei dem Betrieb anfallende Wärme für die Aufheizung des Schrankinnenraumes verwandt. Auch hierdurch wird der Wirkungsgrad des Trockners gesteigert.

Da der Druck im Schrankinnenraum geringer als der Außendruck ist, wird die lediglich angelehnte Schranktür bei Verwendung von Dichtungen durch die Druckdifferenz gegen den Türrahmen gepreßt. Dadurch kann vorteilhafterweise die Tür des Trockenschrankes mit einem Magnetverschluß versehen sein. Ein derartig einfacher Verschluß ist möglich, da die Tür lediglich an der Dichtung anliegen muß. Hierfur sind relativ geringe Kräfte erforderlich. Ein komplizierter Verschlußmechanismus mit Gestängen und Einhakvorrichtungen, wie es bei Schränken mit Innenüberdruck notwendig ist, ist in diesem Fall nicht erforderlich. Hierdurch reduzieren sich ebenfalls die Herstellungskosten.

In einem weiteren Ausführungsbeispiel kann der Trockenschrank eine Metallverkleidung haben, deren einzelne Metallbahnen an ihren Übergängen Löcher aufweisen. Derartige Löcher treten im Zusammenhang mit Paßungenauigkeiten bei der Verlegung der Metallbahnen auf. Da kleine Löcher oder kleine Risse keinen wesentlichen Einfluß auf den Trocknungsvorgang haben, können derartige Undichtigkeiten billigend in Kauf genommen werden, wenn dadurch die Herstellungskosten wesentlich reduziert werden können.

Weiterhin kann die Innenwandung des Trokkenschrankes aus einem wärmeisolierenden Material bestehen. Hierdurch wird auf jeden Fall an den Berührungsstellen zwischen den Textilien und der Schrankinnenwand oder auch der Halterung eine mögliche Gilbbildung an den Textilien unterbunden. Weiterhin ist auch bei empfindlichen Textilien mit Sicherheit auszuschließen, daß eine derartige Materialminderung auftritt. Ebenso verhindert ein solches wärmeisolierendes Material, daß das Bedienungspersonal keine Verbrennungen mehr erleiden kann, wenn nach der Trocknungsphase der Schrank geöffnet wird oder wenn der Trocknungsprozeß unterbrochen ist und das Bedienungspersonal in den noch warmen Schrank faßt.

Um zur Energieeinsparung bei dem erfindungsgemäßen Verfahren auch eine effektive Wärmeisolierung zu erzielen, ist es vorteilhaft, daß die Außenwandung der Zuleitung und der Ableitung des Trockenschrankes aus einem wärmeisolierenden Material besteht. Hierdurch wird ohne einen wesentlichen Energieverlust die erwärmte Luft dem Schrankinnenraum zugeführt. Keine nennenswerte Wärmeenergie tritt aus der Zuleitung in den den Schrank umgebenden Außenraum über. Die aufgewandte Energie wird praktisch ohne Minderung in den Schrankinnenraum überführt. Um die dortigen Temperaturen zu halten, ist es sinnvoll, daß zugleich auch die Innenwandungen aus wärmeisolierendem Material bestehen. Weiterhin gestattet eine Wärmeisolierung des Trockenschrankes an allen Außenflächen, daß ein derartiger Trockenschrank problemlos in Nieschen und neben anderen Aggregaten problemlos einzubauen ist, da ein Wärmeübergang von dem Trockenschrank auf umliegende Gegenstände praktisch unterbunden ist. Hierdurch ist auch nicht erforderlich, die sonst bei Geräten mit hohen Temperaturen erforderlichen Isolierungsabstände zu den anderen umliegenden Gerätschaften einhalten zu müssen. Ein wünschenswerter Platzgewinn ist somit die Folge.

Ein erfindungsgemäßes Ausführungsbeispiel des Trockenschrankes wird in der Zeichnung dargestellt. Es zeigen

Figur 1 einen Längsschnitt durch einen Trokkenschrank mit einer parallel zur Rückwand verlaufenden Schnittebene,

Figur 2 einen Horizontalschnitt auf Höhe des Turbinenrades durch den Trockenschrank der Figur 1 mit einer parallel zum Boden verlaufenden Schnittebene und

Figur 3 einen Horizontalschnitt auf Höhe des Motors durch den Trockenschrank der Figur 1 mit einer parallel zum Boden verlaufenden Schnittebene.

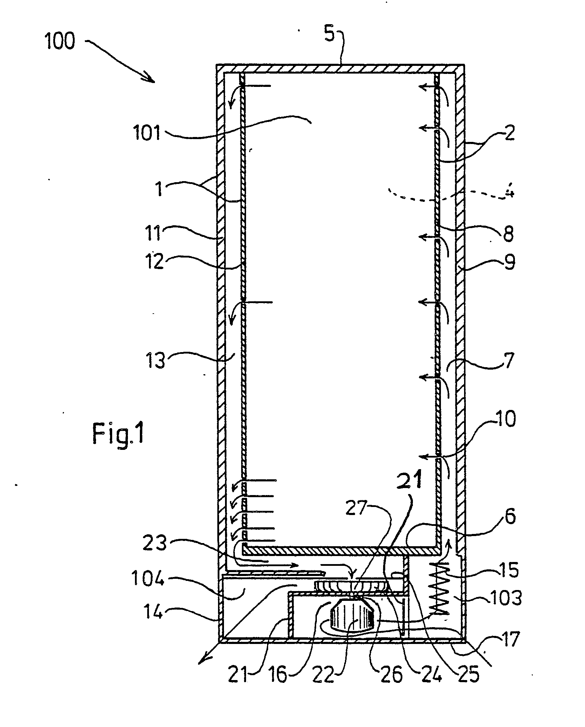

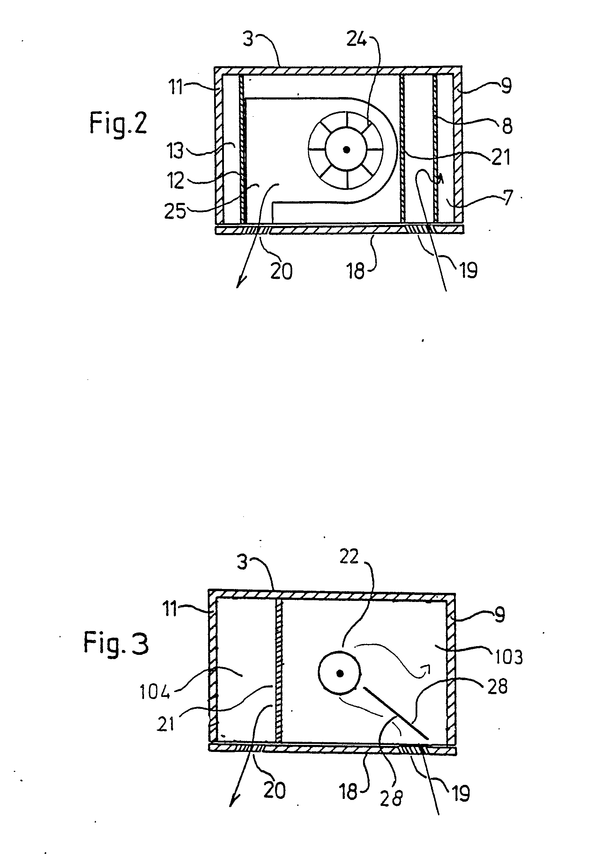

Die Figur 1, 2 und 3 zeigt einen erfindungsgemäßen Trokkenschrank 100 in unterschiedlichen Darstellungen. In seinem Inneren besitzt er einen Schrankinnenraum 101, der seitlich durch doppelwandige Seitenwände 1 und 2, eine Rückwand 3 und eine verschließbare Tür 4 und oben und unten durch eine Decke 5 und einen Innenboden 6 begrenzt ist. Durch die rechte doppelwandige Seitenwand 2 wird eine Zuleitung 7 gebildet, die eine nach innen liegende Zuleitungsinnenwand 8 und eine zum Außenbereich gerichtete Zuleitungsaußenwand 9 besitzt. Die Zuleitungsinnenwand 8 trägt horizontal verlaufende Luftschlitze 10, die eine Verbindung zwischen der Zuleitung 7 und dem Schrankinnenraum 101 herstellen. In den Schrankinnenraum 101 wird das zu trocknende Gut gestellt, gelegt oder mittels einer Aufhängevorrichtung aufgehängt. Die der Zuleitung 7 gegenüberliegende Seitenwand 1 bildet mit einer nach außen gerichteten Ableitungsaußenwand 11 und einer nach innen gerichteten Ableitungsinnenwand 12 eine Ableitung 13, die ebenfalls durch horizontal verlaufende Lüftungsschlitze 10' mit dem Schrankinnenraum 101 verbunden ist.

Unter dem Schrankinnenraum 101 befindet sich ein Sockel 14, in dem sich eine Heizung 15 und ein Gebläse 16 befindet. Nach außen ist der Sockel 14 durch die Rückwand 3, die Fortsetzung der Zuleitungsaußenwand 9 und die Fortsetzung der Ableitungsaußenwand 11, durch einen Außenboden 17 und eine vordere Verblendung 18 abgegrenzt. Die Verblendung 18 trägt zwei Lüftungsgitter, nämlich auf der rechten Seite eine Luftansaugöffnung 19 und auf der linken Seite eine Luftauslaßöffnung 20. Zweckmäßigerweise werden die Lamellen der Öffnungen 19 und 20 schräg angeordnet, so daß die Luft von rechts vorne angesaugt und nach links vorne ausgestoßen wird.

Im Sockel werden zwei Räume, ein Heizraum 103 und ein Gebläseraum 104 durch eine winkelförmig verlaufende Trennwand 21 gebildet. Der Heizraum 103 steht einerseits über die Luftansaugöffnung 19 mit der Außenluft in Verbindung und geht andererseits in die Zuleitung 7 über. In diesem Heizraum 103 befindet sich die Heizung 15 und ein Motor 22 des Gebläses 16. Dabei sorgt ein vertikal ausgerichtetes Luftleitblech 28 dafür, daß die durch die Luftansaugöffnung einströmende Luft zum Teil erst an den Motor 22 vorbeistreift und anschließend zu der Heizung 15 gelangt, um von dort in die Zuleitung 7 überführt zu werden.

Der Gebläseraum 104 steht mit der Ableitung 13 durch einen Kanal 23 in Verbindung, der in dem Zentrum eines Turbinenrades 24 des Gebläses 16 endet. Von dort gelangt bei Betrieb die Luft mittels Zentrifugalkräfte beschleunigt und durch einen Turbinenkäfig 25 gelenkt in den Gebläseraum 104 und wird anschließend durch die Luftauslaßöffnung 20 in die Außenluft ausgestoßen. Bemerkenswert ist, daß das Turbinenrad 24 und der Motor 22 in unterschiedlichen Räumen untergebracht sind. Sie sind jedoch durch eine Welle kraftschlüssig miteinander verbunden, wobei für die Welle in der Trennwand 21 eine Aussparung 27 vorgesehen ist, die zugleich im wesentlichen luftdicht ausgebildet ist.

Die Wände des Schrankes bestehen aus wärmeisolierendem Material, um die zuvor beschriebenen Vorteile in Bezug auf Energieeinsparung und Verhalten gegenüber dem umliegenden Raum in Anspruch zu nehmen.

Bei einem Trockenschrank, bei dem das erfindungsgemäße Verfahren Anwendung findet, wird der Lufttransport durch das Gebläse 16 hervorgerufen, welches in Bezug auf den Luftstrom die Luft erst nach Durchströmen des Schrankinnenraumes 101 aufnimmt und weiterleitet. Die Außenluft wird über die Luftansaugöffnung 19 in den Heizraum 103 gesaugt, wobei das Luftleitblech 28 den Luftstrom erst an dem Motor 22 des Gebläses 16 vorbeiströmen läßt. Anschließend passiert die Luft die Heizung, um die erforderliche und gewünschte Lufttemperatur zu erzielen und gelangt daraufhin in die Zuleitung 7. Durch die horizontal verlaufenden Luftschlitze 10 strömt die Luft aus der Zuleitung 7 in den Schrankinnenraum 101, in dem sich das zu trocknende Gut befindet. Hier wird im Zusammenhang mit dem Trocknungsprozeß Wasser aus seiner flüssigen in seine gasförmige Phase überführt, wobei die an dem Gut vorbeistreichende Luft mit Feuchtigkeit angereichert wird. Diese feuchtere Luft wird anschließend durch die ebenfalls horizontal verlaufenden Luftschlitze 10' an der Ableitungsinnenwand 12 in die Ableitung 13 gesaugt und gelangt von dort zurück in den Sockel. Dabei wird die Luft von der Ableitung 13 durch einen Kanal 23 in das Zentrum des Turbinenrades 24 geführt. Die Drehung dieses Turbinenrades 24 schleudert die Luftmoleküle nach außen, die dort von dem Turbinenkäfig 25 aufgefangen und in den Gebläseraum 104 geleitet werden. Von dort gelangt die Luft durch die Luftauslaßöffnung 20 in den Außenraum, der den Trockenschrank umgibt.

In einem weiteren Ausführungsbeispiel ist es möglich, Teile des Heizraumes 103 und des Gebläseraumes 104 als Wärmeaustauscher im Gegenstrom- oder Parallelstrom-Prinzip auszugestalten.


Bezugszeichenliste:

100 Trockenschrank
101 Schrankinnenraum
103 Heizraum
104 Gebläseraum
1 linke Seitenwand
2 rechte Seitenwand
3 Rückwand
4 Tür
5 Decke
6 Innenboden
7 Zuleitung
8 Zuleitungsinnenwand
9 Zuleitungsaußenwand
10, 10' Luftschlitz
11 Ableitungsaußenwand
12 Ableitungsinnenwand
13 Ableitung
14 Sockel
15 Heizung
16 Gebläse
17 Außenboden
18 Verblendung
19 Luftansaugöffnung
20 Luftauslaßöffnung

21 Trennwand
22 Motor
23 Kanal
24 Turbinenrad
25 Turbinenkäfig
26 Welle
27 Aussparung
28 Luftleitbleche

**Ansprüche**

1. Verfahren zum Trocknen, Lüften und Sterilisieren von Gut, wie von Wäsche, besonders empfindlichen Geweben, reiner Wolle, Oberbetten, Kissen, Daunendecken, Wolldecken und Leder, festes Gut wie medizinische Instrumente, Bestecke, Kämme, Scheren, Bürsten, Messer und Rasierapparate, in einem Trockenschrank mit einem die Luft bewegenden Gebläse,
wobei Außenluft durch eine Heizung erwärmt wird, danach einen Schrankinnenraum mit darin befindlichem Gut durchströmt und anschließend aus dem Trockenschrank durch eine Luftauslaßöffnung ausströmt,
dadurch gekennzeichnet,
daß das Gebläse (16) die Luft aus dem Trockenschrank (100) absaugt.

2. Verfahren nach Anspruch 1, dadurch gekennzeichnet, daß durch Veränderung der Förderleistung des Gebläses (16) bei konstanter Leistung der Heizung (15) unterschiedliche Durchflußmengen an Luft durch den Trockenschrank (100) gesaugt werden.

3. Trockenschrank zur Durchführung der Verfahren nach einem der Ansprüche 1 oder 2,
mit einer Luftansaugöffnung, mit einer von der Luftansaugöffnung kommenden, zu dem Schrankinnenraum führenden Zuleitung, wobei eine Heizung für die Trockenluft innerhalb der Zuleitung angeordnet ist, und mit einer von dem Schrankinnenraum wegführenden und mit einer Luftauslaßöffnung verbundenen Ableitung,
dadurch gekennzeichnet,
daß das Gebläse (16) innerhalb der Ableitung (13) angeordnet ist.

4. Trockenschrank nach Anspruch 3, wobei das Gebläse aus auf einer gemeinsamen Welle liegendem Motor und Turbinenrad besteht, dadurch gekennzeichnet,
daß Motor (22) und Turbinenrad (24) durch eine im wesentlichen luftdichte Wand (21) getrennt sind, die eine Durchführung für die Welle (26) besitzt.

5. Trockenschrank nach einem der Ansprüche 3 oder 4,
dadurch gekennzeichnet,
daß die Tür (2) des Trockenschrankes (100) mit einem Magnetverschluß versehen ist.

6. Trockenschrank nach einem der Ansprüche 3 bis 5,
dadurch gekennzeichnet,
daß der Trockenschrank (100) eine Metallverkleidung hat, deren einzelne Metallbahnen an ihren Übergängen Löcher aufweisen.

7. Trockenschrank nach einem der Ansprüche 3 bis 5,
dadurch gekennzeichnet,
daß die Innenwandungen (1; 2; 3; 4; 5; 6; 8 und 12) des Trockenschrankes (100) aus einem wärmeisolierenden Material bestehen.

8. Trockenschrank nach einem der Ansprüche 3 bis 5,
dadurch gekennzeichnet,
daß die Außenwandungen (9 und 11) der Zuleitung (7) und der Ableitung (13) des Trockenschrankes (100) aus einem wärmeisolierenden Material bestehen.

Fig.1

Fig.2

Fig.3